# EUROPEAN PATENT APPLICATION

(11) **EP 1 226 818 A1**
(43) Date of publication of application: **31.07.2002**
(21) Application number: 02250201.7
(22) Date of filing: 11.01.2002
(51) Int. Cl.: A61K 9/00, A61K 9/16, A61K 9/20

(54) **Pharmaceutical dosage forms with enhanced cohesive and compressibility properties**

(30) Priority: 26.01.2001 US 264378 P
(71) Applicant: Pfizer Products Inc., Groton, Connecticut 06340 (US)
(72) Inventor: Vendola, Thomas Anthony, Groton, Connecticut 06340 (US)
(74) Representative: Hayles, James Richard

(57) **Abstract**

Methods and granules for preparing pharmaceutical dosage forms that are formed by compression and other compressed devices having enhanced cohesive and compressibility properties. The method comprises forming a first granule comprising a solid pharmaceutically acceptable volatilizable agent and an active ingredient, volatizing the solid volatilizable agent from the first granule to form a second granule and compressing the second granule to form a compressed device.

## Description

### BACKGROUND OF THE INVENTION

This invention relates to methods for preparing pharmaceutical dosage forms that are formed by compression and other compressed devices having enhanced cohesive and compressibility properties.

U.S. Patent No. 3,885,026 discloses a process for the production of porous tablets using a solid volatilizable adjuvant that is incorporated in the tablets. After the tablets are formed by compression, the volatilizable adjuvant is removed by sublimation or thermal decomposition.

U.S. Patent No. 4,134,943 discloses the production of porous tablets by mixing the tablet components with a liquid solvent which is inert towards the tablet components to form a mixture that is subsequently divided into small particles or droplets and frozen. The frozen granules are pressed into tablets at a temperature below the freezing point of the solvent and then the solvent is sublimed from the tablets.

U.S. Patent Nos. 4,305,502 and 4,371,516 disclose the production of shaped articles by freezing in a mold a water-based pharmaceutical composition and subliming the water from the frozen composition to form porous articles.

Commonly assigned U.S. Patent No. 5,516,530 discloses a method for making porous shaped delivery devices by compressing a frozen pharmaceutical formulation containing a beneficial agent, a water soluble polymer and water so as to locally liquefy, shape and refreeze the formulation and lyophilizing the formulation to form a porous shaped delivery device.

Commonly assigned U.S. Patent No. 5,529,789 discloses a method for making high strength, highly porous fast dissolving delivery devices comprising mixing a formulation comprising menthol, a water-soluble, menthol soluble polymer, and an active agent at a temperature such that the menthol is substantially molten, disposing the formulation in a mold, solidifying the formulation and subliming the solidified molded formulation.

Commonly assigned U.S. Patent No. 5,853,758 discloses a method for making porous tablets by combining and compressing ingredients of a tablet that include a meltable binder, together with a suitable volatilizable component such as menthol, camphor, urea, vanillin or ammonium bicarbonate, melting and solidifying the binder and then volatizing the volatilizable component to form a porous tablet.

Pharmaceuticals in the form of solid shaped tablets are typically manufactured by compressing the materials that make up the final product into the desired tablet form. Such materials may include active pharmaceutical ingredients as well as pharmaceutically non-active "excipients" that impart necessary or useful properties to the product during and after the manufacturing process. Excipients may include any of a multitude of different types of materials, including diluents, lubricants, glidants, disintegrants, coloring and flavoring agents, coating materials, binders and compressive agents.

Tablet dosage forms may include a class of excipients known as compressive agents. A tablet must have sufficient cohesive and/or compressive properties so that its powdered components will remain intact from the time following compression until the time of administration. Such cohesive or compressive properties may be provided by active pharmaceutical ingredients themselves or by excipients in the dosage form. Compressive agents may be added to impart such properties.

Tablet hardness, or tensile strength can be used as a measure of the cohesiveness of the ingredients of a tablet. If a tablet does not posses sufficient cohesive properties the tablet may fall apart on handling.

There are various processes used by those skilled in the art for preparing tablet dosage forms. Examples of such processes include wet-granulation, dry-granulation, fluid-bed granulation and direct compression. The type of method used may depend upon factors such as physical characteristics of the active pharmaceutical ingredients in the formulation, the types of excipients used and the desired physical characteristics of the final product. Each of these processes include steps involving mixing of the ingredients of the dosage form.

Some amount of mixing of the ingredients of a dosage form is usually necessary in order to have a homogeneous and consistent final product. However, in the preparation of pharmaceutical tablets by wet and dry granulation it has been found that the extent and intensity of the mixing of the ingredients prior to compression is related to a loss of compressibility and cohesiveness of the formulation, resulting in reduced tablet hardness.

A similar result may be observed when roller compaction is used, for example, in dry granulation methods. Roller compaction may be employed as a method to form the granules that are subsequently compressed into tablets. Roller compaction may reduce the subsequent compressibility and cohesiveness of the dosage form.

### SUMMARY OF THE INVENTION

One aspect of this invention is methods for preparing a pharmaceutical dosage form comprising:
forming first granules comprising a solid pharmaceutically acceptable volatilizable agent and a pharmaceutically active ingredient by a granulation method selected from a wet granulation method and a dry granulation method;
volatizing the solid volatilizable agent from the first granules to form second granules;
compressing the second granules to form a pharmaceutical dosage form.

Another aspect of this invention is pharmaceutical granules having enhanced compressive properties prepared by a method comprising:
forming first granules comprising a solid pharmaceutically acceptable volatilizable agent and a pharmaceutically active ingredient by a granulation method selected from a wet granulation method and a dry granulation method; and
volatilizing said volatilizable agent from the first granules to form pharmaceutical granules.

A further aspect of this invention is methods for preparing a compressed device comprising:
forming first granules comprising a solid volatilizable agent and an active ingredient by a granulation method;
volatizing the solid volatilizable agent from the first granules to form second granules;
compressing the second granules to form a compressed device.

In a preferred embodiment of this invention, the volatilizable agent is selected from menthol, camphor, urea, vanillin, urethane, hexamethylene tetramine, benzoic acid, phthalic anhydride, naphthalene, ammonium bicarbonate, solid water, solid cyclohexane and solid tert-butyl alcohol, more preferably, menthol, camphor, urea, vanillin, and ammonium bicarbonate and even more preferably, ammonium bicarbonate.

In another preferred embodiment of this invention, the volatilizable agent comprises about five percent to about 20 percent of said first granules by weight.

In a further preferred embodiment of this invention, the first granules are formed by a granulation method selected from a wet granulation method and a dry granulation method and, more preferably, a wet granulation method.

In an additional preferred embodiment of this invention, the first granules further comprise a compressive agent, more preferably a pharmaceutically acceptable compressive agent. Compressive agents that are preferred include starch, sucrose, mannitol, dextrose, lactose, dicalcium phosphate, and cellulosic materials, including carboxymethylcellulose, methylcellulose, hydroxypropylmethylcellulose, hydroxyethylcellulose, hydroxypropylcellulose, microcrystalline cellulose and silicified microcrystalline cellulose, more preferably microcrystalline cellulose and silicified microcrystalline cellulose.

The term "compressive agents" means an agent added to the ingredients of a compressed device such as a pharmaceutical dosage form prepared by compression, which imparts cohesive and/or compressive properties to such ingredients. The term compressive properties as used herein describes the relationship in a particular substance between the compression of the substance and the hardness or tensile strength of the resulting compressed device.

The terms "granule" and granules as used herein mean an agglomeration or agglomerations of the particles of the ingredients of a compressed device such as a pharmaceutical dosage form prepared by compression. A granule may contain all of the ingredients of the compressed device or only some of the ingredients.

### BRIEF DESCRIPTION OF THE DRAWINGS

Figure 1 is a comparison of the effect of variations in the mixing time on tablet hardness according to Example 1 of the Experimental Procedures.

Figure 2 is a comparison of tablet hardness between a formulation made with a volatilizable agent and a formulation containing no volatilizable agent according to Example 2 of the Experimental Procedures.

Figure 3 is a comparison of differences in tablet hardness resulting from variations in the treatment of ammonium bicarbonate used to prepare such formulations according to Example 3 of the Experimental Procedures.

### DETAILED DESCRIPTION OF THE INVENTION

The ingredients in the pharmaceutical dosage form aspects of the present invention include at least one pharmaceutically active ingredient, a volatilizable agent and, optionally, one or more additional excipients.

Any volatilizable agent, preferably a pharmaceutically acceptable volatilizable agent, may be used which is readily sublimable from a solid or crystalline state or which readily converts into gaseous decomposition products from a solid or crystalline state and which are compatible with the other components of the pharmaceutical composition. Suitable volatilizable agents include menthol, camphor, urea, vanillin, urethane, hexamethylene tetramine, benzoic acid, phthalic anhydride, naphthalene and ammonium bicarbonate. Water, cyclohexane and tert-butyl alcohol which are liquid at ambient temperature, but which can be sublimed from their solid or crystalline state, are also suitable volatilizable agents. Other volatilizable agents will be apparent to those skilled in the art.

The volatilizable agent is incorporated into the pharmaceutical mixture before, during or at the end of mixing of the ingredients of the pharmaceutical dosage form and prior to compression of the ingredients into a tablet-like device. For example, the volatilizable agent can be combined with all or some of the ingredients and then mixed to form the pharmaceutical mixture in the form of granules that will be compressed into tablets. Alternatively, the ingredients are mixed without the volatilizable agent, which is added at the very end of the mixing process.

A correlation is observed between tablet hardness and the degree of intimacy in the mixture between the volatilizable agent and the other ingredients of a pharmaceutical composition. As the intimacy of the mixture is increased, so does the hardness of the tablet increase. Therefore, it is preferable that the pharmaceutical mixture be prepared such that the intimacy in the mixture between the volatilizable agent and the other ingredients of the pharmaceutical formulation is enhanced.

For volatilizable agents that are incorporated as solids, increased intimacy may be achieved by reducing the particle size of the agent. A reduced particle size may be obtained by screening the agent through a wire mesh or through milling.

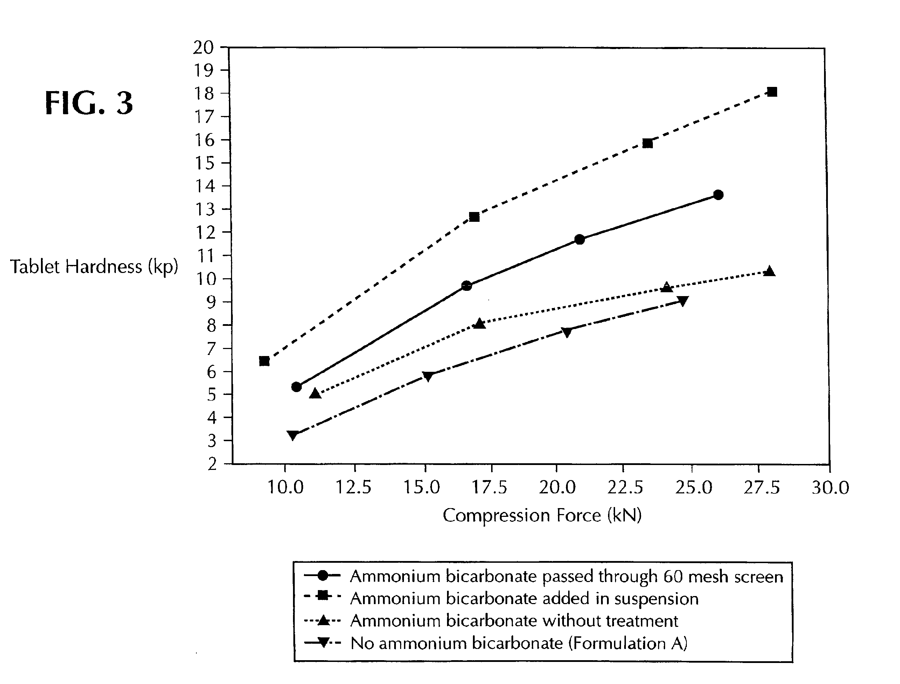

Greater intimacy may also be achieved by incorporating the agent in a liquid as a suspension or solution before applying it to the pharmaceutical composition. Figure 3 in the Experimental Procedures exemplifies the increase in tablet hardness resulting from increasing the mixture intimacy between a volatilizable agent and the other components of a pharmaceutical mixture.

The quantity of volatilizable agent that is effective to improve tablet cohesiveness and compressibility is between about one percent and about 40 percent by weight of the total pharmaceutical composition, and preferably between about five percent and about 20 percent of the total composition.

In addition to the active pharmaceutical ingredients and volatilizable agents, any of a number of excipients may be used in pharmaceutical compositions of this invention. Remington: The Science and Practice of Pharmacy, Mack Publishing Company, Easton, Pa., 19th Edition 1995 provides a number of excipients that are well known by those skilled in the art. The selection of excipients will depend on the properties that are to be imparted to the dosage form and the properties possessed by the active pharmaceutical ingredient.

For example, a compressive agent may, if necessary, be used to impart additional compressibility and/or cohesiveness to the materials of a dosage form or compressed device. Examples of compressive agents include starch, sucrose, mannitol, dextrose, lactose, dicalcium phosphate, and cellulosic materials, including carboxymethylcellulose, methylcellulose, hydroxypropylmethylcellulose (HPMC), hydroxyethylcellulose (HEC), hydroxypropylcellulose (HPC), microcrystalline cellulose (MCC), such as Avicel PH101®, and silicified microcrystalline cellulose (SMCC), such as Prosolv 50®. However, no compressive agent may be necessary if the active ingredient(s) together with any other ingredient(s) of the dosage form possess sufficient properties of compression and cohesiveness.

Other excipients that may be useful or desirable for particular pharmaceutical compositions in tablet form include diluents, lubricants, glidants, disintigrants, coloring and flavoring agents, coating materials and binders. Those skilled in the art will appreciate that selection of any of these other excipients will also depend on the properties that are to be imparted to the dosage form and the properties already possessed by the other ingredients of the dosage form.

The pharmaceutically active ingredient in the invention may be any compound that is pharmaceutically active on oral, rectal or vaginal administration. Examples of such compounds include antifungal agents such as fluconazole, pain relievers such as acetaminophen and acetylsalicylic acid, antihistamines such as diphenhydramine, doxylamine succinate and meclizine, decongestants such as pseudoephedrine hydrochloride, antibiotics such as azithromycin and erythromycin, penicillins such as sultamicillin tosylate and amoxicillin trihydrate, enzyme inhibitors such as sulbactam sodium, antihypertensives such as nifedipine, doxazosin mesylate and amlodipine besylate, antidiabetics such as glipizide, bronchodilators such as pirbuterol hydrochloride and theophylline, anti-inflammatory agents such as piroxicam and celecoxib, antidepressants such as sertraline hydrochloride, antacids such as calcium carbonate and magnesium oxide, non-sedative antihistamines such as cetirizine, phosphodiesterase type 5 (PDE5) inhibitors such as sildenafil citrate, cholesterol reducing agents, such as atorvastatin calcium and anti-viral agents such as nelfinavir mesylate. Pharmaceutically active agents can include nutritional and dietary supplements, for example, vitamins.

Wet granulation methods may be employed for preparing the granules of the pharmaceutical composition. Wet granulation methods are described in Remington: The Science and Practice of Pharmacy, Mack Publishing Company, Easton, Pa., 19th Edition 1995. These and other methods are generally known by those skilled in the art. If wet granulation is employed, the volatilizable agent is incorporated in the mixture before, during or after mixing of the ingredients, but prior to formation of granules. For example, a solid volatilizable agent can be blended with the powders of the mixture prior to, during or after the addition of binding agent solutions. Alternatively, the volatilizable agent can be a component of the binding agent solutions as a suspension or dissolved therein. Mixing of the ingredients may be performed using methods known by those skilled in the art, including twin shell blending as well as high shear mixing using, for example, a double cone blender.

Dry granulation methods may also be employed for preparing the pharmaceutical dosage form. Reminaton, ibid., provides a description of dry granulation methods. These and other methods are generally known by those skilled in the art. As in wet granulation, the volatilizable agent is incorporated in the dry granulation mixture before or during mixing of the ingredients and before granulation of the ingredients.

Volatilization of the volatilizable agent is performed prior to compression of the pharmaceutical mixture into a tablet-like dosage form or compressed device. Volatilization may be performed by any method that results in sublimation or gaseous decomposition of the volatilization agent in its solid state. It is preferable that the volatilization methods employed have no detrimental effect on the pharmaceutical composition or its components.

Volatilization may be accomplished by the application of vacuum or heat, or a combination of both vacuum and heat. Alternatively, volatilization may be performed at ambient temperature and pressure. The method of volatilization used depends upon the physical properties of the volatilization agent, characteristics of the pharmaceutical formulation and practical manufacturing considerations.

An important aspect of the invention is that the volatilizable agent be in solid form at the time of volatilization. It will be apparent to those skilled in the art that for volatilizable agents that are liquid at ambient temperature, the temperature of the volatilizable agent and the pharmaceutical mixture must be maintained below the freezing point of the volatilizable agent during the volatilization of the volatilizable agent. It will also be apparent to those skilled in the art that for such volatilizable agents that are added in solid form, the temperature of the volatilizable agent and the pharmaceutical mixture must be maintained below the freezing point of the volatilizable agent during the mixing and volatilization steps.

For example, certain volatilizable agents such as water, cyclohexane and tert-butyl alcohol that are liquids at ambient temperature can be incorporated into the pharmaceutical mixtures as liquids and subsequently frozen and removed by lyophilization. Alternatively, these volatilizable agent may be incorporated into the mixtures as solids (i.e., by maintaining the agent at a temperature below its freezing point) and then removed under vacuum, with or without the application of heat.

Other volatilizable agents, such as menthol, are solid at ambient temperature. Such volatilizable agents may be incorporated into the pharmaceutical mixtures as solids and subsequently volatilized. Alternatively, solid volatilizable agents may be incorporated with the pharmaceutical composition while in their molten state as liquids and subsequently allowed to solidify prior to volatilization.

If wet granulation methods of tablet preparation are used, the volatilization of the volatilizable agent may be performed during and together with the drying of the granules. For example, if the volatilization is performed by the use of vacuum (e.g., lyophilization using vacuum) or heating it may be possible to employ the same process for drying and for volatilization. Alternatively, the volatilization may be performed after the granules have been formed and dried.

The final step in the formation of the dosage form is compression. Compression may be performed by any method known in the art, including use of a press-type tableting machine which compresses the dosage form between two punches.

This invention also may be applied in the preparation of non-pharmaceutical tablet-type compressed devices for use by consumers and industry. Such applications of the invention are particularly useful for the preparation of devices that require mixing of ingredients followed by compression into devices wherein such mixing results in a loss of cohesiveness and compressibility of the ingredients. Examples of non-pharmaceutical applications include the preparation of compressed pool chlorination tablets, cleaning, deodorizing and/or disinfection tablets such as for use in toilets, flavored candies and other food items formed into compressed tablets, soaps and detergents in the form of compressed tablets and multi-colored writing chalk formed by compression.

### EXPERIMENTAL PROCEDURES

The present invention is illustrated by the following examples, but is not limited to the details thereof.

The ingredients of Formulation A and Formulation B are provided solely for illustrative purposes. It will be appreciated by those skilled in the art that the selection of ingredients in a formulation will depend on the physical characteristics of the active pharmaceutical ingredient(s) used as well as the properties that are desired in the final product.

| Formulation A - Tablets Made Without Volatilizable Agent | |
|---|---|
| **Intra-Granular Components** | **mg/Tablet** |
| Active pharmaceutical ingredient | 126.74 |
| Microcrystalline Cellulose | 107.26 |
| Mannitol | 45.00 |
| Hydroxypropyl Cellulose | 3.00 |
| Croscarmellose Sodium | 7.50 |
| TOTAL | 289.50 |

| **Extra-Granular Component** | **mg/Tablet** |
|---|---|
| Croscarmellose Sodium | 7.50 |
| Colloidal Silicone Dioxide | 1.50 |
| Magnesium Stearate | 1.50 |
| TOTAL | 300.00 |

| Formulation B - Tablets Made With Ammonium Bicarbonate (10%) | |
|---|---|
| **Intra-Granular Component** | **mg/Tablet** |
| Active pharmaceutical ingredient | 126.74 |
| Microcrystalline Cellulose | 107.26 |
| Mannitol | 45.00 |
| Ammonium Bicarbonate | 30.00 |
| Hydroxypropyl Cellulose | 3.00 |
| Croscarmellose Sodium | 7.50 |
| TOTAL | 319.50 |

| **Extra-Granular Component** | **mg/Tablet** |
|---|---|
| Croscarmellose Sodium | 7.50 |
| Colloidal Silicone Dioxide | 1.50 |
| Magnesium Stearate | 1.50 |
| TOTAL | 330.00* |

| | |
|---|---|
| * Volatization of ammonium bicarbonate reduces final weight to 300 mg | |

### General Formulation Method

Both Formulation A and Formulation B are prepared by the same general method. Wet granulation mixing of the intra-granule components is performed in a Niro-Fielder SP-1 high shear granulator (GEA Niro Inc., 9165 Rumsey Road, Columbia, MD 21045) with the impeller set at 600 rpm and the chopper set at 2000 rpm. The wet granules are made with a water content of 25%. Mixing time varies between 3 and 20 minutes depending on the sample desired. The wet granules are dried overnight in a forced hot air oven at 60°C. The drying step also serves to volatilize ammonium bicarbonate in those granule samples that contain it. The resulting dried granules are passed through a Comil, model 197, (Quadro Inc, 55 Bleeker St, Millbum, NJ 07041) at an impeller speed of 1380 rpm using a 2A-1601-173 impeller and a 2A-055R037/32 screen. The extra-granule components are blended with the dried granules in a twin shell blender (Patterson-Kelly, 100 Burson St, East Stroudsburg, PA). Granules are compressed using a Kilian T-100 (Kilian & Co, 415 Sargon Way Unit I, Horsham, PA) rotary tablet press using a compression force in the range of approximately 8 to approximately 30 kN.

The tablet hardness in Examples 1, 2 and 3, was measured using a Schleuniger tablet tester (Schleuniger Pharmatron Inc, Manchester, NH). The hardness of a tablet is the force in kilopounds (kp) required to break a tablet. The higher the kp value, the stronger is the tablet.

### EXAMPLE 1

### Comparison of the Effect of Variations in Mixing Time on Tablet Hardness.

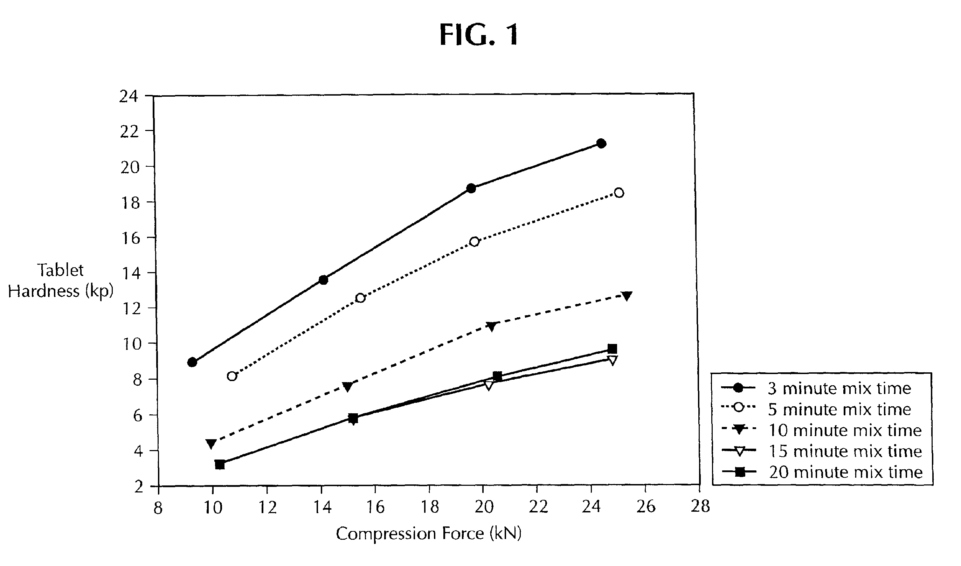

Tablets were prepared using Formulation A. Batches were prepared with varying mixing times of 3 minutes, 5 minutes, 10 minutes, 15 minutes and 20 minutes. Each batch was used to prepare tablets using four separate levels of compression force. The effect of different mixing times is illustrated in Figure 1.

### EXAMPLE 2

### Comparison of Tablet Hardness - Formulation A vs. Formulation B.

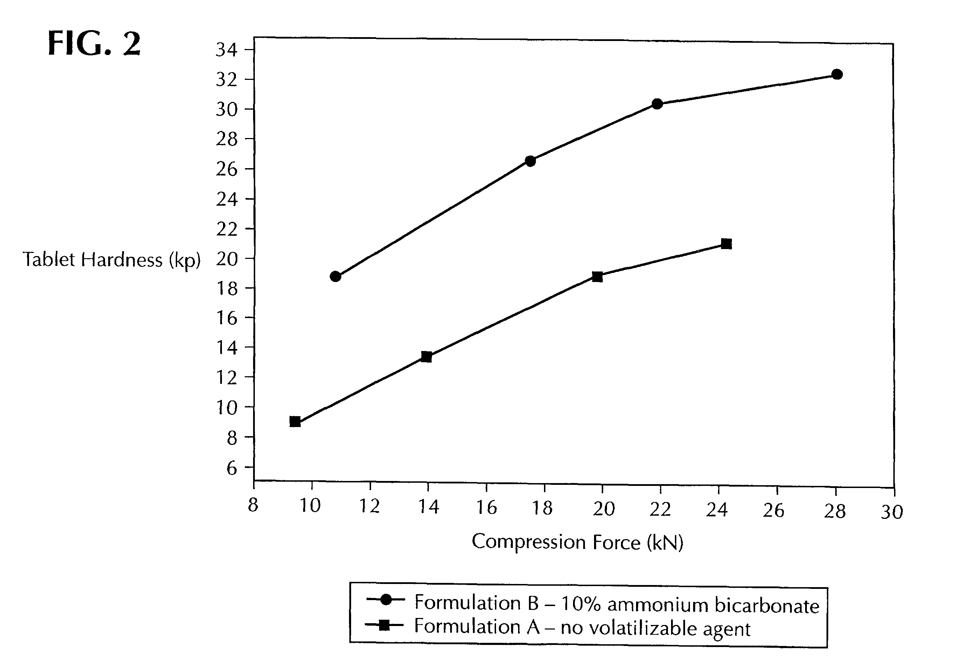

Tablets were prepared using Formulation A, containing no volatilizable agent, and Fomulation B, containing 10% ammonium bicarbonate. All batches were prepared using a mixing time of 3 minutes. Tablets were prepared using four separate levels of compression force. The comparison of the two formulations is illustrated in Figure 2.

### EXAMPLE 3

### Comparison of Differences in Tablet Hardness Resulting from Treatment of Ammonium Bicarbonate.

Tablets were prepared using Formulation A, containing no volatilizable agent, and Formulation B, containing 10% ammonium bicarbonate. All batches were prepared using a mixing time of 20 minutes. Batches of the Formulation B tablets were made with untreated ammonium bicarbonate and ammonium bicarbonate treated as follows:
(1) Ammonium bicarbonate was passed by hand through a 60 mesh sieve (VWR Scientific Products, 1310 Goshen Pkwy, West Chester, PA).
(2) Ammonium bicarbonate in suspension was first milled by passing through a Fitz Mill model JT Comminutor (The Fitzpatrick Company, 832 Industrial Drive, Elmhurst IL) at high speed (5000 rpm) using an N000 screen with hammers forward. The suspension was made by adding the ammonium bicarbonate to water using a stir plate.

The camparison of the various methods of treating ammonium bicarbonate is illustrated in Figure 3.

## Claims

1. A method for preparing a pharmaceutical dosage form comprising:
forming first granules comprising a solid pharmaceutically acceptable volatilizable agent and a pharmaceutically active ingredient by a granulation method selected from a wet granulation method and a dry granulation method;
volatizing the solid volatilizable agent from the first granules to form a second granule;
compressing the second granules to form a pharmaceutical dosage form.

2. A method of claim 1 wherein said granulation method is a wet granulation method.

3. A method of claim 1 or 2 wherein said first granules further comprise a pharmaceutically acceptable compressive agent.

4. A method of claim 1-3 wherein said volatilizable agent is selected from menthol, camphor, urea, vanillin, urethane, hexamethylene tetramine, benzoic acid, phthalic anhydride, naphthalene, ammonium bicarbonate, solid water, solid cyclohexane and solid tert-butyl alcohol.

5. A method of claim 4 wherein said volatilizable agent is selected from menthol, camphor, urea, vanillin, and ammonium bicarbonate.

6. A method of claim 5 wherein said volatilizable agent is ammonium bicarbonate.

7. A method of claim 1-6 wherein the volatilizable agent comprises about five percent to about 20 percent of said first granules by weight.

8. Pharmaceutical granules prepared by a method comprising:
forming first granules comprising a solid pharmaceutically acceptable volatilizable agent and a pharmaceutically active ingredient by a granulation method selected from a wet granulation method and a dry granulation method; and
volatilizing said volatilizable agent from the first granules to form pharmaceutical granules.

9. Pharmaceutical granules of claim 8 wherein said granulation method is a wet granulation method.

10. Pharmaceutical granules of claim 8 or 9 wherein said first granules further comprise a pharmaceutically acceptable compressive agent.

11. Pharmaceutical granules of claim 8-10 wherein said volatilizable agent is selected from menthol, camphor, urea, vanillin, urethane, hexamethylene tetramine, benzoic acid, phthalic anhydride, naphthalene, ammonium bicarbonate, solid water, solid cyclohexane and solid tert-butyl alcohol.

12. Pharmaceutical granules of claim 11 wherein said volatilizable agent is ammonium bicarbonate.

13. A method for preparing a compressed device comprising:
forming first granules comprising a solid volatilizable agent and an active ingredient;
volatilizing the solid volatilizable agent from the first granules to form second granules;
compressing the second granules to form a compressed device.

14. A method of claim 13 wherein said volatilizable agent is selected from menthol, camphor, urea, vanillin, urethane, hexamethylene tetramine, benzoic acid, phthalic anhydride, naphthalene, ammonium bicarbonate, solid water, solid cyclohexane and solid tert-butyl alcohol.

15. A method of claim 14 wherein said volatilizable agent is ammonium bicarbonate.
